# EUROPÄISCHE PATENTSCHRIFT

(11) **EP 2 654 641 B1**
(45) Veröffentlichungstag und Bekanntmachung des Hinweises auf die Patenterteilung: **14.12.2016**
(21) Anmeldenummer: 11831798.1
(22) Anmeldetag: 20.12.2011
(51) Int. Cl.: A61M 1/00, A61F 13/02, A61F 13/10, A61F 13/06, A61F 15/00

(54) **ABDECKELEMENT MIT EINEM MANSCHETTENKÖRPER**
COVERING ELEMENT WITH A SLEEVE BODY
ÉLÉMENT COUVRANT COMPORTANT UN CORPS MANCHON

(30) Priorität: 23.12.2010 DE 102010056019; 23.12.2010 DE 102010056018
(43) Veröffentlichungstag der Anmeldung: 30.10.2013
(73) Patentinhaber: Erdmann, Alfons, 51429 Bergisch Gladbach (DE)
(72) Erfinder: Erdmann, Alfons, 51429 Bergisch Gladbach (DE)
(74) Vertreter: Fischer, Uwe
(86) Internationale Anmeldenummer: PCT/DE2011/050060
(87) Internationale Veröffentlichungsnummer: WO 2012/083934

(56) Entgegenhaltungen:
- GB-A- 2 237 508
- US-A1- 2010 174 250
- US-B2- 7 350 785

## Beschreibung

Die Erfindung liegt auf dem Gebiet der Fertigungstechnik und der Medizintechnik und bezieht sich auf ein Abdeckelement für den gasdichten Abschluss eines Körperteils mit einem Folienelement.

Die Erfindung ist außer im medizinischen Sektor auch auf anderen Gebieten einsetzbar, bei denen Teile gasdicht eingeschlossen werden sollen, insbesondere dann, wenn ein Teil eines gasdicht abzuschließenden Elementes aus dem Abdeckelement herausragt. Dies kann beispielsweise bei längeren strangartigen Körpern wie Rohren oder dergleichen der Fall sein, wenn ein Teil eines Rohres gasdicht abgeschlossen werden muss.

Für den gasdichten Abschluss liegt es nahe, ein flexibles Element, beispielsweise eine Folie zu verwenden, die partiell um den abzuschließenden Körper herum gewickelt wird.

Ein Problem stellt dabei insbesondere der gasdichte Abschluss an den Grenzflächen zwischen dem Abdeckelement und dem einzuschließenden/abzuschließenden Körper dar, sowie das gasdichte Zusammenfügen verschiedener Teile des Abdeckelementes untereinander.

Noch anspruchsvoller wird die Aufgabe, wenn es darum geht, ein solches Abdeckelement zu schaffen, das auch problemlos wieder entfernbar ist.

Der vorliegenden Erfindung liegt in diesem Zusammenhang die Aufgabe zugrunde, ein möglichst kostengünstiges einfach aufgebautes Abdeckelement für den gasdichten Abschluss eines Körperteils/eines Bauelementes zu schaffen, das zuverlässig einen gasdichten Abschluss schaffen kann und möglichst einfach wieder entfernbar ist.

Die Aufgabe wird gemäß der Erfindung mit den Merkmalen des Patentanspruchs 1 gelöst.

Dazu ist zusätzlich zu einem Folienelement ein Manschettenkörper vorgesehen, der als Prisma mit drei spitzwinklig aufeinander stoßenden Seitenflächen ausgebildet ist.

Dieser Manschettenkörper ermöglicht das Ausfüllen von Zwickelräumen, die entstehen wenn das Folienelement um den Körper herum angeordnet und an diesem gedichtet wird. Dort wo zwei Teile des Folienelementes um den abzuschließenden Körper herum zusammentreffen, ergibt sich ein Zwickelraum zwischen dem Körper einerseits und den beiden zusammentreffenden Folienelementen andererseits. An dieser Stelle kann vorteilhaft der Manschettenkörper eingesetzt werden, der auf den abzuschließenden Körper/das Körperteil aufgelegt wird und das faltenfreie Anschmiegen der verschiedenen Teile des Folienelementes erlaubt, ohne dass das Folienelement stark geknickt werden muss. Es ergibt sich damit eine wesentliche Verbesserung der Gasdichtigkeit des Abschlusses.

Der Manschettenkörper kann einerseits vorgefertigt sein, er kann jedoch auch vor Ort dadurch gebildet sein, dass ein verformbarer Stoff beim Zusammenfügen des Folienelementes eingelegt und damit zu einem Manschettenkörper oder zu einem Teil eines Manschettenkörpers geformt wird. Der verformbare Stoff kann beispielsweise ein Gel sein, das in einer stabilisierten Gelform weiter verbleibt oder sich nachfolgend verfestigt. Es sind auch andere Materialien denkbar, wie Kunststoffe, die durch temperatur- oder Strahlungseinwirkung bei der Anwendung verfestigbar sind oder sebsttätig härten, wie beispielsweise übliche Klebstoffe.

Besonders vorteilhaft kann dabei vorgesehen sein, dass wenigstens eine der Seitenflächen im Querschnitt des Prismas konkav ausgebildet ist.

Damit können die Außenkonturen des Prismas/Manschettenkörpers sich noch glatter an den abzuschließenden Körper, insbesondere das abzuschließende Körperteil anschmiegen und auch dort, wo die verschiedenen Teile des Folienelementes zusammentreffen, laufen sie derart spitzwinklig aufeinander zu, dass eine zwischenraumfreie Abdichtung möglich wird.

Die Herstellung des Manschettenkörpers aus einem geschlossenporigen Schaumstoff macht diesen leicht und einfach handhabbar sowie in gewissem Umfang verformbar, wobei dennoch die Gasdichtigkeit garantiert ist.

Der Manschettenkörper kann vorteilhaft mit einem Kleber beschichtet sein, um ein gasdichtes Haften verschiedener Teile des Folienelementes und/oder des gasdicht abzuschließenden Körpers zu erleichtern.

Vorteilhaft kann dabei vorgesehen sein, dass die Beschichtung an den Kanten, an denen die Seitenflächen des jeweiligen Prismas aneinander angrenzen, über die Kanten hinaus ragt.

Hierdurch wird auch dort, wo drei verschiedene Elemente wie beispielsweise der abzuschließende Körper, der Manschettenkörper und das Folienelement aneinander anliegen, das Entstehen von die Dichtigkeit gegebenenfalls beeinträchtigenden Zwischenräumen verhindert. Der über die Kanten des Manschettenkörpers hinausragende Kleber ist vorteilhaft gelartig plastisch verformbar beziehungsweise fließfähig, haftet jedoch andererseits tropffrei an dem Manschettenkörper.

Wenigstens einer der Manschettenkörper weist vorteilhaft eine gasdichte Durchführung für Fluide auf, die insbesondere als Durchführung für einen Gasschlauch zum Absaugen von Gas ausgebildet sein kann. In diesem Fall kann nach dem Abdichten des Abdeckelementes durch den Manschettenkörper hindurch Gas oder ein anderes Fluid zugeführt oder abgezogen werden, insbesondere kann der durch das Abdeckelement abgeschlossene Innenraum evakuiert werden.

Es kann zudem vorgesehen sein, dass der Gasschlauch an seinem Ende innerhalb des durch das Abdeckelement abgeschlossenen Innenraums mantelseitige Öffnungen aufweist.

In diesem Fall kann der Gasschlauch an seinem Ende sackartig verschlossen sein und beim Absaugen von Gas kann dies ausschließlich durch die mantelseitigen Öffnungen am Ende des Gasschlauches angesaugt werden. Dieses Ende wird dann vorteilhaft in ein Granulat oder einen offenporigen Körper beziehungsweise ein offenes Gewebe im Bereich einer Wunde eines abzuschließenden Körperteils gebracht, so dass dort im wundnahen Bereich Vakuum erzeugt und damit die Heilung begünstigt werden kann.

Eventuell dort vorhandene Sekrete können ebenfalls mit abgesaugt werden.

Das Vorhandensein einer Vielzahl von mantelseitigen Öffnungen in dem Schlauch verhindert, dass durch das Verstopfen einzelner Öffnungen das Absaugen von Fluid unmöglich wird.

Die Erfindung bezieht sich außer auf ein Abdeckelement in der oben beschriebenen Form auch auf einen Manschettenkörper, der zur Verwendung bei einem derartigen Abdeckelement einsetzbar ist. Entsprechende Abdeckelemente bestehen vorteilhaft aus flexiblen Folien. Eine solche Folie, aus der ein Folienelement gebildet ist, kann beispielsweise auch aus einem geschlossenporigen oder offenporigen Schaumstoff, ein- oder mehrschichtig ausgeführt, bestehen.

Die Erfindung wird im folgenden anhand eines Ausführungsbeispiels in einer Zeichnung gezeigt und anschließend beschrieben.

Dabei zeigt
Figur 1 in einer dreidimensionalen Ansicht ein Folienelement;
Figur 2 in einem Querschnitt ein um ein Körperteil herum angeordnetes Folienelement;
Figur 3 eine Draufsicht auf einen Ausschnitt eines Folienelementes mit Vorfixierelementen;
Figur 4 ein Detail im Querschnitt mit zwei Passelementen;
Figur 5 eine dreidimensionale Ansicht eines Manschettenkörpers als Durchführung für eine Vakuumverbindung;
Figur 6 eine spezielle Ausführungsform eines Manschettenkörpers in dreidimensionaler Ansicht;
Figur 7 eine Draufsicht auf einen Ausschnitt eines Folienelementes mit einem Abziehelement;
Figur 8 ein Abziehelement in einer Draufsicht;
Figur 9 einen Querschnitt durch ein Abziehelement;
Figur 10 einen Querschnitt durch ein Abziehelement in teilweise abgezogener Position;
Figur 11 einen Querschnitt durch eine andere Variante eines Abziehelementes,
Figur 12 eine dreidimensionale Ansicht eines schlauchförmigen Abziehelementes in teilweise abgezogener Position,
Figur 13 schematisch ein Körperteil, das auf einem Folienelement 1 angeordnet ist,
Figur 14 ein vorbereitetes Vlies aus einem offenporigen Schaumstoff, der als flache Folie ausgebildet ist
Figur 15 in einer dreidimensionalen Darstellung einen Absaugkörper mit rechteckigem Querschnitt
Figur 16 ebenfalls eine dreidimensionale Ansicht eines Absaugkörpers,
Figur 17 in einer Seitenansicht einen Teil des Manschettenkörpers im Längsschnitt,
Figur 18 perspektivisch und schematisch ein Körperteil, das auf einem Abdeckelement in Form einer Schaumstofffolie liegt,
Figur 19 eine Ansicht eines Abdeckelementes mit Abziehelementen, die einen umlaufenden Streifen von aufgetragenem Kleber vollständig abdecken,
Figur 20 schematisch den Zustand, in dem ein Klebstreifen teilweise zur Vorfixierung des Körperteils freigelegt ist,
Figur 21 eine Anordnung eines Körperteils, um das herum ein Abdeckelement hochgezogen ist,
Figur 22 eine Anordnung wie aus Figur 21, wobei das Abdeckelement bereits an einem Manschettenkörper anliegt,
Figur 23 eine Seitenansicht der Anordnung aus Figur 22, wobei ein Ende eines Abziehelements seitlich schräg aus dem Abdeckelement herausragt, um weiter abgezogen werden zu können,
Figur 24 eine Seitenansicht eines Körperteils 2, in diesem Fall eines Beins, mit einem Abdeckelement 1,
Figur 25 einen Abziehstreifen beziehungsweise ein Paar von Abziehstreifen in detaillierterer Form, sowie
Figur 26 einen einzelnen doppellagigen Abziehstreifen in einer Draufsicht.
Figur 27, ein erfindungsgemäßes Flachmaterial;
Figur 28, ein Flachmaterial, das eine Rinnenform aufweist;
Figur 29, ein Flachmaterial, das im Gegensatz zu Figur 28 auf seiner Unterseite eine Rinne aufweist;
Figur 30, die Ausführungsform aus Figur 29 mit einer auf einer ebenen Unterlage flachgedrückten Rille;
Figur 31, ein aus zwei unterschiedlichen Lagen bestehendes Flachmaterial,
Figur 32, im Querschnitt eine gasdichte röhrenförmige Abdeckung in einem Querschnitt im Bereich der abzudeckenden Extremität;
Figur 33, einen Querschnitt durch ein Abschlusselement;
Figur 34, eine Seitenansicht einer Abdeckung, die einen Abschnitt an einem Bein abdeckt;
Figur 35, eine Seitenansicht einer Abdeckung, die das untere Ende eines Beins mitsamt einem Fuß abdeckt;
Figur 36, eine dreidimensionale Ansicht der Ausgestaltung gemäß Figur 35,
Figur 37, dreidimensional eine Ausgestaltung der gasdichten Abdeckung für eine Hand und einen Unterarm, sowie
Figur 38, dreidimensional einen Abschlusskörper mit einem Absatz.

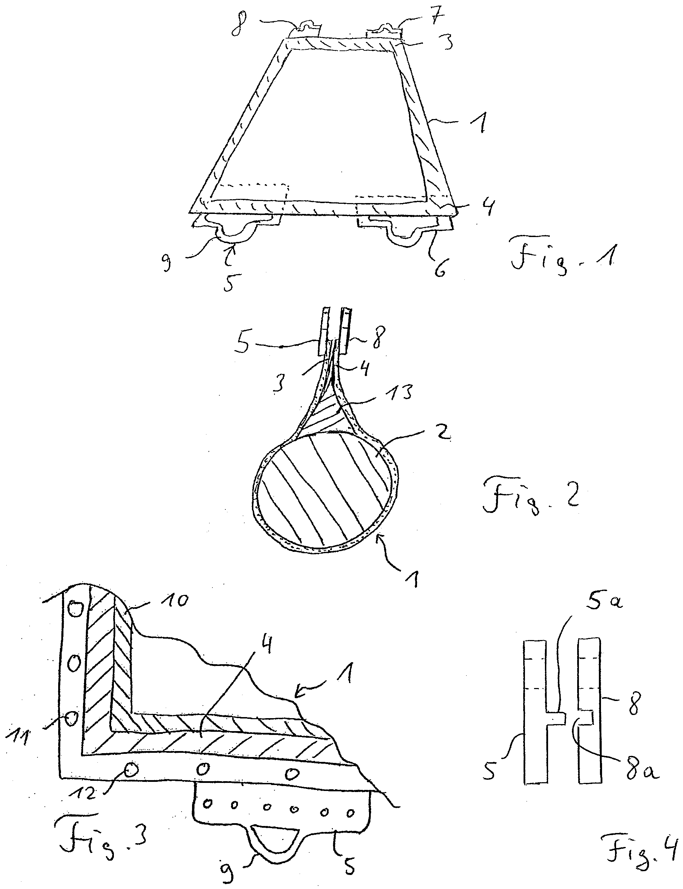

Figur 1 zeigt in einer dreidimensionalen Ansicht ein Folienelement 1, das als Flachmaterial die Ausgangsform für ein Abdeckelement bildet, rechteckig geformt ist und beispielsweise aus zwei Schichten bestehen kann. Ein derartiges Folienelement dient beispielsweise zum gas- oder vakuumdichten Verpacken eines Körperteils um bei bestimmten Heilverfahren das Umfeld einer offenen Wunde gasdicht verschließen beziehungsweise evakuieren zu können. Zu diesem Zweck wird ein Körperteil, das in der Figur 2 mit dem Bezugszeichen 2 bezeichnet ist, auf das Folienelement 1 gelegt und dieses zu beiden Seiten hochgeschlagen, wobei die Passelemente 5, 6, 7, 8 zur Deckung gebracht werden sollten, um eine symmetrische Anordnung des Folienelementes 1 um das Körperteil herum zu erzielen.

Das Folienelement kann aus zwei Folienschichten bestehen, die voneinander trennbar sind, um im inneren Bereich eine Folie vorzusehen, die gasdicht und anschmiegsam ist, um sich vakuumdicht um das abzuschließende Körperteil herum zu legen und in dem übrigen Bereichen vakuumdicht zusammenfügbar zu sein. Die äußere Folienschicht kann beispielsweise aus einer etwas steiferen Schicht bestehen, die nur zur Handhabung der inneren, flexibleren Folienschicht dient und später entfernt werden kann. Zudem schützt die stabilere äußere Folienschicht die innere Folienschicht vor Verletzungen. Die verschiedenen Schichten können aber auch dauerhaft und untrennbar miteinander verbunden sein.

Ein derartiges Folienelement ist vorteilhaft mit Fügeflächen 3,4 versehen, die umlaufend am Rand des Folienelementes verteilt sind und beispielsweise mit einem Kleber vorbeschichtet sein können, um auf dem einzuschließenden Körperteil gasdicht zu haften oder um eine Haftung von Teilen des Folienelementes an anderen Teilen des Folienelementes in gasdichter Form zu erzielen.

Naturgemäß ist das Folienelement schwierig zu handhaben, wenn die Fügeflächen frei liegen. Zu diesem Zweck sind die Fügeflächen mit Abziehelementen abgedeckt, die auf den Fügeflächen abziehbar haften. Auf diese wird weiter unten detaillierter eingegangen.

In der Figur 1 sind an den Rändern des Folienelementes 1 Passelemente 5, 6, 7, 8 dargestellt, die an der Außenseite des Folienelementes aufgesetzt sind und auch abnehmbar sein können. Diese weisen jeweils Handgriffe 9 auf, die das Folienelement im praktischen Einsatz gut handhabbar machen.

Die Figur 2 zeigt im Querschnitt das Körperteil 2, das der Einfachheit halber als im Querschnitt runde Säule dargestellt ist und das darum herum geschlungene Folienelement 1. An den Enden des Folienelementes 1 sind zwei Passelemente 5, 8 dargestellt, die deckungsgleich aneinander anliegen.

Zur besseren Dichtung des Folienelementes an dem Körperteil 2 ist ein Manschettenkörper 13 in Form eines Prismas vorgesehen, der in dem Zwickel zwischen dem Körperteil 2 und den zusammenzufügenden Rändern/Fügeflächen 3, 4 des Folienelements 1 liegt.

Das Vorsehen des Manschettenkörpers 13 gewährleistet für das Folienelement 1 ein deutlich einfacheres und zuverlässigeres Anschmiegen und Haften in dem Bereich des Überganges von dem Körperteil 2 zum Manschettenkörper 13 und am Ende des Manschettenkörpers 13, an dem die Fügeflächen des Folienelementes 1 zusammentreffen.

In der Figur 3 ist ein Detail des Folienkörpers 1 dargestellt, wobei eine mit Klebstoff versehene Fügefläche 4 zu erkennen ist sowie ein Passelement 5 mit einem Handgriff 9.

Um eine gute Vorfixierung des Folienelementes 1 vor dem Zusammenfügen der Fügeflächen 3, 4 zu erreichen, sind Vorfixierelemente 10, 11, 12 vorgesehen, die beispielsweise als Klettelemente ausgebildet sein können. Dabei stellt 10 einen Klettstreifen dar, während 11 und 12 Klettpunkte /pads darstellen. An Stelle etwaiger Klettflächen können auch leicht lösbare und vorteilhaft gegeneinander verschiebbare Haftbeläge vorgesehen sein.

Gelingt es daher, die Fügeflächen 4 vor dem Zusammenfügen abzudecken, so kann das Folienelement 1 zunächst anhand der Paßelemente und der Vorfixierelemente in eine optimale Position gebracht werden, um danach die Fügeflächen frei zu legen und ein festes Haften der Fügeflächen aneinander in gasdichter Form zu erzielen. Die Passelemente können innerhalb der Fügeflächen im später gasdicht abgeschlossenen Raum oder außerhalb der Fügeflächen vorgesehen sein.

In der Figur 4 ist in einem Querschnitt die Gestaltung der Passelemente 5, 8 detaillierter gezeigt, wobei das Passelement 5 Zapfen 5a aufweist, die in Passlöcher 8a des Passelementes 8 hineinpassen und damit nach dem Zusammenfügen der Passelemente die verschiedenen Teile des Folienelementes 1 gegeneinander optimal formschlüssig positionieren. Zapfen und Paßlöcher können auch ineinander rasten und nur durch Anwenden einer bestimmten Mindestlösekraft voneinander lösbar sein.

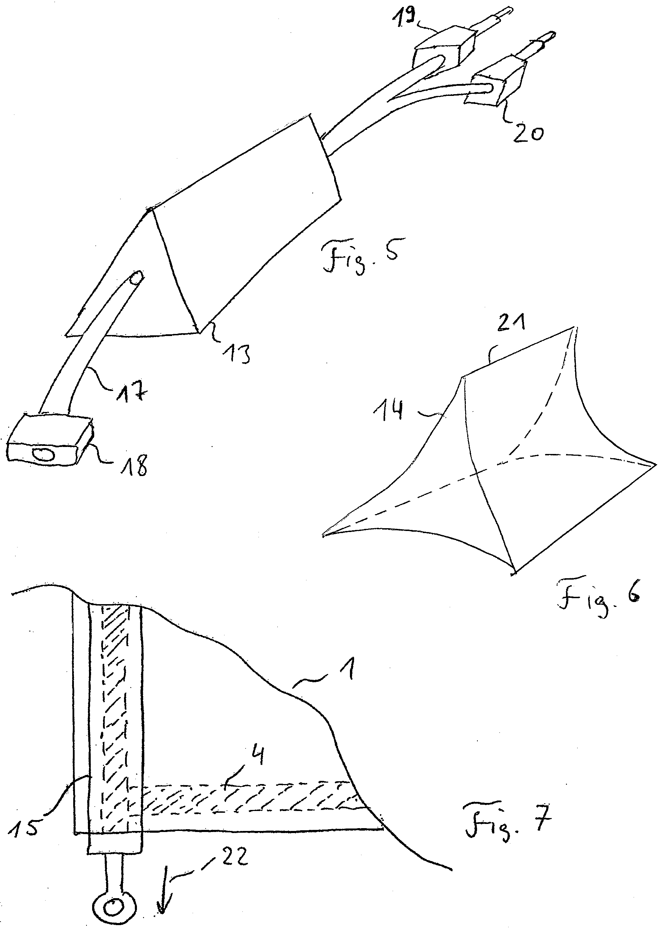

Die Figur 5 zeigt in dreidimensionaler Darstellung einen Manschettenkörper 13, der vorteilhaft aus einem geschlossenporigen Schaumstoff besteht und beispielsweise durch Laser- oder Wasserstrahlschneiden aus einem Schaumstoffblock gewonnen wird. Der prismenförmige Manschettenkörper 13 bietet damit eine glatte Oberfläche in seinem Mantelbereich, an der die Fügeflächen 3, 4 des Folienelementes 1 optimal und gasdicht haften. Die Mantelflächen des Manschettenkörpers 13 können zudem mit einem Klebstoff vorbeschichtet sein, um die Abdichtung und Haftung weiter zu optimieren.

In der Figur 5 ist eine Vakuumleitung 17 gezeigt, die mittels eines Vakuumconnectors 18 zu einem Absauganschluss führt. Die Vakuumleitung 17 ist durch den Manschettenkörper 13 gasdicht durchgeführt und verzweigt auf der anderen Seite des Manschettenkörpers 13, die später im abgeschlossenen Innenraum des Folienelementes liegt, in zwei Anschlüsse 19, 20, die weiter zu Saugleitungen an zwei verschiedenen, innerhalb des abgeschlossenen Bereichs liegenden Wunden an dem eingeschlossenen Körperteil führen können. Die Enden derartiger Saugleitungen sind als sackartig abgeschlossene Schlauchenden mit mehreren mantelseitigen porenartigen Saugöffnungen ausgebildet.

Diese werden im Wundbereich innerhalb eines Granulats oder eines offenporigen Schaumstoffs oder eines sonstigen durchlässigen Stoffs angeordnet, durch den der Wundbereich evakuiert und gegebenenfalls auch Wundsekrete abgesaugt werden können.

Die Figur 6 zeigt einen Manschettenkörper 14, der mit einem Klebstoff 21 bedeckt ist, der im Bereich der Mantelfläche über die Kanten des Manschettenkörpers 14 ein Stück weit hinaus gezogen ist um im Zwickelbereich zwischen dem Manschettenkörper und dem Folienelement 1 eine plastische oder fließende Verformung des Klebers zur vollständigen Abdichtung zu erlauben. Der Kleber kann dazu eine gelartige Konsistenz aufweisen.

Zudem ist der prismenförmige Manschettenkörper 14 mit im Prismenquerschnitt konkaven Seitenflächen ausgebildet, um im Bereich der Zwickelräume zwischen dem Manschettenkörper einerseits, dem Folienelement 1 andererseits und beispielsweise einem einzuschließenden Körperteil winkelmäßig bezüglich der Oberflächenkontur im Übergangsbereich den gasdichten Abschluss zu optimieren.

In der Figur 7 ist ein Detail eines Folienelementes 1 dargestellt, wobei eine Fügefläche 4 teilweise zu sehen ist. Eine weitere Fügefläche ist durch ein darauf liegendes Abziehelement 15 verdeckt. Die Abziehrichtung und Längsrichtung des Abziehelementes ist durch einen Pfeil 22 gekennzeichnet.

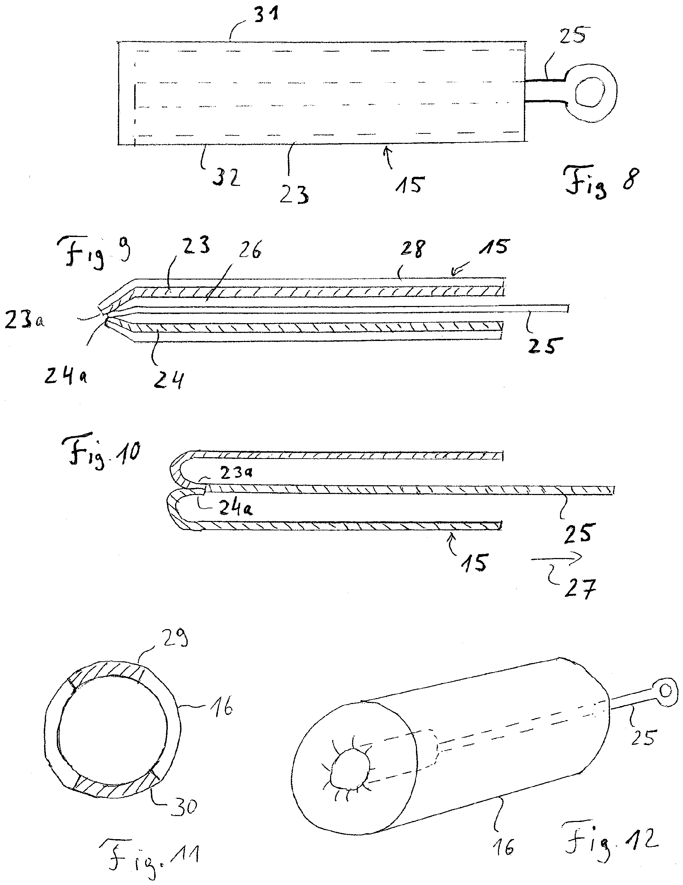

In der Figur 8 ist ein Abziehelement 15 genauer dargestellt. Dabei ist ein Haftstreifenelement 23 dargestellt sowie ein Zugelement 25.

Das Zugelement 25 verläuft wie in Figur 9 deutlicher zu erkennen zwischen dem ersten Haftstreifenelement 23 und einem deckungsgleich darunter liegenden zweiten Haftstreifenelement 24 in deren Zwischenraum 26. An den Enden 23a, 24a der Haftstreifenelemente 23, 24 ist das Zugelement 25 mit diesen verbunden. Zudem können die beiden Enden 23a, 24a auch miteinander verbunden sein.

In der Figur 10 ist die Funktion des Abziehelementes 15 schematisch erkennbar dargestellt. Die Enden 23a, 24a der Haftstreifenelemente werden durch Zug an dem Zugelement 25 in Längsrichtung des Abziehelementes (Richtung des Pfeils 29) in den Zwischenraum 26 zwischen den Haftstreifenelementen 23, 24 hineingezogen.

Es findet dadurch eine Umstülpbewegung der Haftstreifenelemente statt, in deren Zuge das Äußere des Abziehelementes 15 gewissermaßen nach innen gezogen wird. Dadurch findet eine Einrollbewegung der Haftstreifenelemente 23, 24 statt, wobei diese, wenn sie zwischen zwei Fügeflächen angeordnet sind, von diesen abgerollt und entfernt werden, ohne dass sie gegenüber den Fügeflächen in einer Scherbewegung verschoben werden müssten.

Auf diese Weise kann das Abziehelement, selbst wenn es fest zwischen zwei Fügeflächen 3, 4 eines zusammengelegten Folienelementes 1 nach dessen Vorfixierung oder zwischen einer Fügefläche 3,4 und einer als Fügefläche fungierenden Hautbereich des gasdicht abzuschließenden Körperteils liegt, mit geringer Kraft herausgezogen werden. Dadurch kommen die Fügeflächen 3, 4 abstandslos aufeinander zu liegen und fügen sich fest und gasdicht zusammen.

Um ein besseres Ablösen der Haftstreifenelemente 23, 24 im Zuge der Einrollbewegung von den Fügeflächen zu erzielen, können die Haftstreifenelemente zusätzlich mit einer Silikonschicht 28 versehen sein. Die Haftstreifenelemente können jedoch auch ohnedies aus einem Stoff hergestellt sein, der auf den Fügeflächen schlecht haftet.

In der Figur 11 ist eine andere Variante eines Abziehelementes dargestellt, bei der zwei Haftstreifenelemente 29, 30 als Teile eines Schlauches 16 ausgebildet sind, der abgeplattet zwischen zwei Fügeflächen liegen kann. Wird an dem Zugelement 25 gezogen, so wird dieser Schlauch, wie in Figur 12 ersichtlich, wie ein Strumpf umgestülpt.

Zudem ist das Zugelement 25 im Inneren des Schlauchs 16 zuverlässig geführt.

Um einen ähnlichen Effekt bei flächenartigen Haftstreifenelementen 23, 24 zu erreichen, können diese in ihren Randbereichen 31, 32 auch miteinander verbunden sein, vorteilhaft in lösbarer Form, beispielsweise durch Verkleben, Verschweißen oder Umbördeln.

In diesem Fall wird sukzessive, in dem Maß, in dem die Haftstreifen sich von dem Fügeflächen lösen und in den Zwischenraum 26 gezogen werden, auch die randseitige Verbindung zwischen den Haftstreifen gelöst. Die randseitige Verbindung bewirkt jedenfalls eine sichere Führung des Zugelementes, so dass dieses nicht seitlich zwischen den Haftstreifen heraus gleiten kann.

Das Folienelement 1 besteht beispielsweise aus einer inneren Folie, die aus Polyurethan bestehen kann, während die äußere Folienlage des Folienelementes 1 beispielsweise aus Polyethylen bestehen kann. Es sind hier jedoch auch andere Materialpaarungen möglich.
Besonders vorteilhaft besteht das Folienelement in allen dargestellten Ausführungsbeispielen aus einem gasdicht ausgeführten Schaumstoff, der entweder geschlossenporig oder offenporig mit einer gasdichten Schicht gestaltet ist. Die gasdichte Schicht kann beispielsweise auf der Außen- oder Innenseite des Folienelementes vorgesehen sein. Sie kann durch Beschichtung des Schaumstoffes oder durch einen Prozess erzeugt sein, durch den die Poren geschlossen werden. Der Manschettenkörper 13 besteht vorteilhaft aus einem Polyurethanschaumstoff, der geschlossenporig ausgeführt ist, um die Vakuumdichtigkeit zu gewährleisten.

Die Haftstreifenelemente oder allgemein Abziehelemente können vorteilhaft aus Polyethylen oder aus einer Verbindung aus Polyethylen und Polypropylen bestehen und silikonisiert sein.

Die Passelemente 5, 6, 7, 8 können aus einem festen Kunststoff bestehen, der lediglich zur Vermeidung von Verletzungen nicht scharfkantig sein sollte.

Als Kleber zur Beschichtung der Fügeflächen 3, 4 wird vorteilhaft ein Acrylatkleber verwendet.

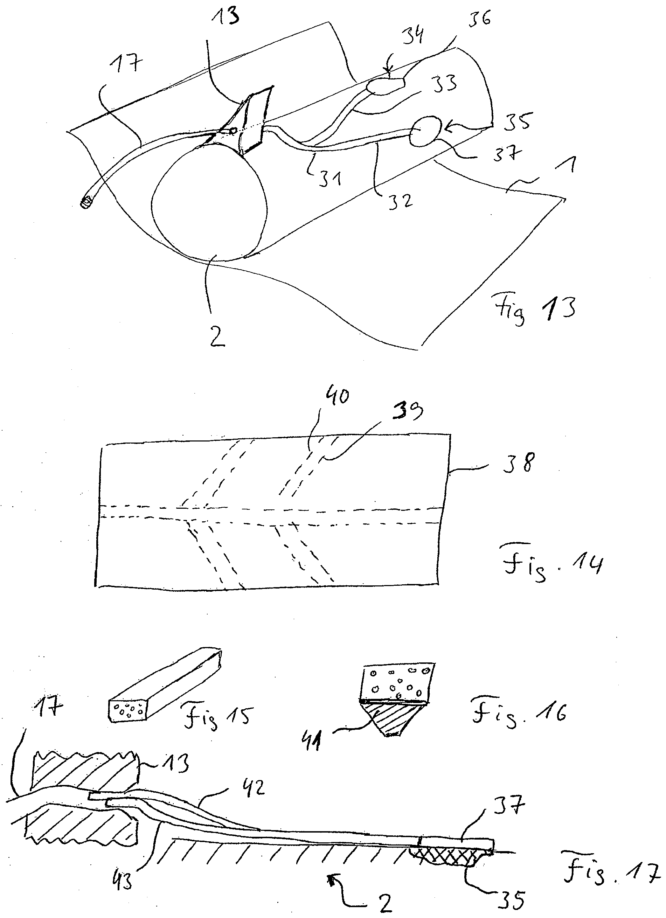

Figur 13 zeigt schematisch ein Körperteil 2, das auf einem Folienelement 1 angeordnet ist, um durch dieses abgedeckt zu werden. Außerdem ist ein Manschettenkörper 13 zur Abdichtung vorgesehen, durch den eine Vakuumleitung 17 hindurchgeführt ist.

Auf der Innenseite des abzudeckenden Volumens ist auf der Oberseite des Körperteils 2 ein Absaugkörper 31 schematisch dargestellt, der aus zwei streifenförmigen Elementen 32, 33 besteht, die, ausgehend von einem einzigen Streifen im Bereich des Manschettenkörpers 13, sich zu zwei Wundbereichen 34, 35 hin verzweigen. Die Wundbereiche 34, 35 sind jeweils mit Abdeckkissen 36, 37 abgedeckt, an oder in denen die Absaugkörper 32, 33 enden.

Die einzelnen Absaugkörper 32, 33 sind als flache Streifen aus einem porösen, offenporigen nachgiebigen Schaumstoff gebildet, die aus einem flachen Vlies bedarfsgerecht ausgeschnitten sind. Sie können einstückig zusammenhängen und gemeinsam ausgeschnitten sein.

Der Absaugkörper ist im Bereich der Durchführung durch den Manschettenkörper 13 in die Öffnung, in der die Vakuumleitung 17 mündet, eingeführt und dort beispielsweise verklemmt.

Die Absaugkörper 32, 33 können derart ausgeschnitten werden, dass sie geometrisch passend von dem Manschettenkörper 13 zu den entsprechenden Wundbereichen 34, 35 führen und nicht verdreht oder gedehnt werden müssen.

Die Figur 14 zeigt ein vorbereitetes Vlies aus einem offenporigen Schaumstoff, das als flache Folie ausgebildet ist und aus dem Absaugkörper beziehungsweise Absaugstreifen passend ausgeschnitten werden können, beispielsweise mit einer Schere.

In dem vorbereiteten Vlies 38 können auch Reißlinien 39, 40 in verschiedenen Formen vorbereitet sein als Perforierungen oder Einstanzungen, die es ermöglichen, auch ohne den Gebrauch eines Schneidwerkzeugs bestimmte Geometrien durch Ausreißen von Teilflächen zu erzeugen.

Figur 15 zeigt in einer dreidimensionalen Darstellung einen Absaugkörper aus offenporigem Schaumstoff mit rechteckigem Querschnitt.

Figur 16 zeigt ebenfalls eine dreidimensionale Ansicht eines Absaugkörpers, wobei dessen Unterseite 41, mit der er auf dem Körperteil 2 aufliegen soll, derart verschlossen oder mit einer geschlossenen gasdichten Schicht bedeckt ist, dass in diesem Bereich keine Unterdruckanwendung stattfindet. Damit wird die Reizung des Gewebes, auf dem der Absaugkörper aufliegt, vermieden.

Figur 17 zeigt in einer Seitenansicht einen Teil des Manschettenkörpers 13 im Längsschnitt mit der Vakuumleitung 17, die in den Manschettenkörper 13 mündet sowie zwei Absaugkörper 42, 43, die in der Öffnung im Manschettenkörper 13 münden und dort eingeklemmt sind. Die Absaugkörper 42, 43 verlaufen von dort zur Oberfläche des Körperteils 2 und dort weiter bis zu einem Wundbereich 35 und einem hier nicht dargestellten weiteren Wundbereich 34, wo die Absaugkörper in Absaugkissen 37 münden. Die Absaugkissen können entweder auch aus einem offenporigen Schaumstoff oder aus einem Granulat oder aus einem offenen Gewebe oder ähnlichem bestehen.

Die Verwendung derartiger, beispielsweise mit einem Granulat gefüllter Absaugkissen, die auf oder in die Wunde eingebracht werden, ist allgemein in Verbindung mit Vakuumverbänden, also gasdichten flachen oder röhrenförmigen Abdeckelementen und entsprechenden Vorrichtungen zur Evakuierung des Wundbereichs sinnvoll. Derartige Kissen können jeweils eine durchlässige Aussenhülle und darin ein Granulat, beispielsweise in Form eines Schüttgutes aufweisen.

Die Absaugkissen können beispielsweise auch fest mit den Absaugkörpern verbunden sein oder im Zuge des Anlegens des Abdeckkörpers verbunden werden.

Die Absaugkörper können auch als Schaumstoffkörper, entweder geschlossenporig oder offenporig ausgebildet sein, wenn in ihrem Inneren eine durchgehende Öffnung, beispielsweise in Form eines Schlauchs oder nur als Ausnehmung, oder als Absaugpfad im offenporigen Schaumstoff vorgesehen ist, die dann von der Vakuumleitung 17 bis zum Absaugkissen 37 führt. Die Ummantelung des entsprechenden Absaugpfades durch nachgiebigen Schaumstoff stellt die Vermeidung von Druckstellen an dem Körperteil 2 sicher.

Es ist auch möglich, die Absaugkörper als schlauchartige Hülle auszubilden, die einerseits verformbar ausgebildet, jedoch andererseits mit einem Granulat zur Stützung gegen ein Einfallen bei Unterdruck gefüllt sind.

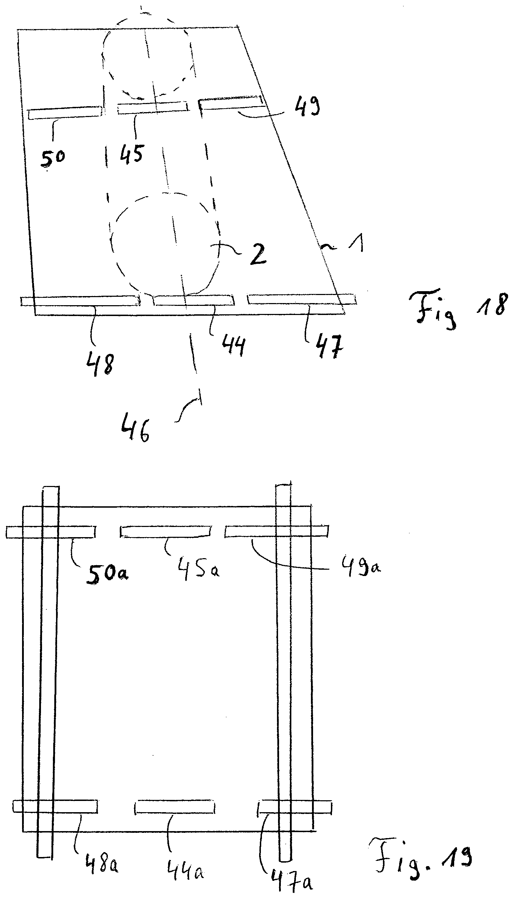

Die Figur 18 zeigt perspektivisch und schematisch ein Körperteil 2, das auf einem Abdeckelement 1 in Form einer Schaumstofffolie liegt, um demnächst abgedeckt zu werden.

Um die Praktikabilität des Anlegevorgangs eines Abdeckelementes bei einem Patienten zu verbessern, ist vorgesehen, dass bei dem Abdeckelement 1 zunächst ein Teilbereich 44, 45 der quer verlaufenden Klebestreifen im mittleren Bereich bezüglich der Längsachse 46 des Abdeckelementes, die parallel zur Längsachse des Körperteils liegen soll, durch Entfernen von Abziehelementen freigelegt wird, damit dort das Körperteil 2 auf dem Abdeckelement 1 abgelegt und bereits vorfixiert werden kann. Übrige Bereiche von vorgesehenen Klebe- und Abdichtverbindungen, die in der Figur 18 schematisch dargestellt und mit 47, 48, 49, 50 bezeichnet sind, bleiben zunächst durch Abziehelemente abgedeckt, damit diese Bereiche noch nicht kleben. Die Aufteilung der Abziehemente 44a, 45a, 47a, 48a, 49a, 50a ist in der Figur 19 dargestellt.

Zwischen den Abziehelementen 48a und 44a, zwischen 44a und 47a, zwischen 50a und 45a und zwischen 45a und 49a sind jeweils Unterbrechungen oder zumindest Sollbruchstellen oder Sollreißstellen vorgesehen, die es in einfacher Weise ermöglichen, im mittleren Bereich entsprechende Abziehelemente 44a, 45a getrennt und unabhängig von den übrigen Abziehelementen in einem ersten Schritt zu entfernen.

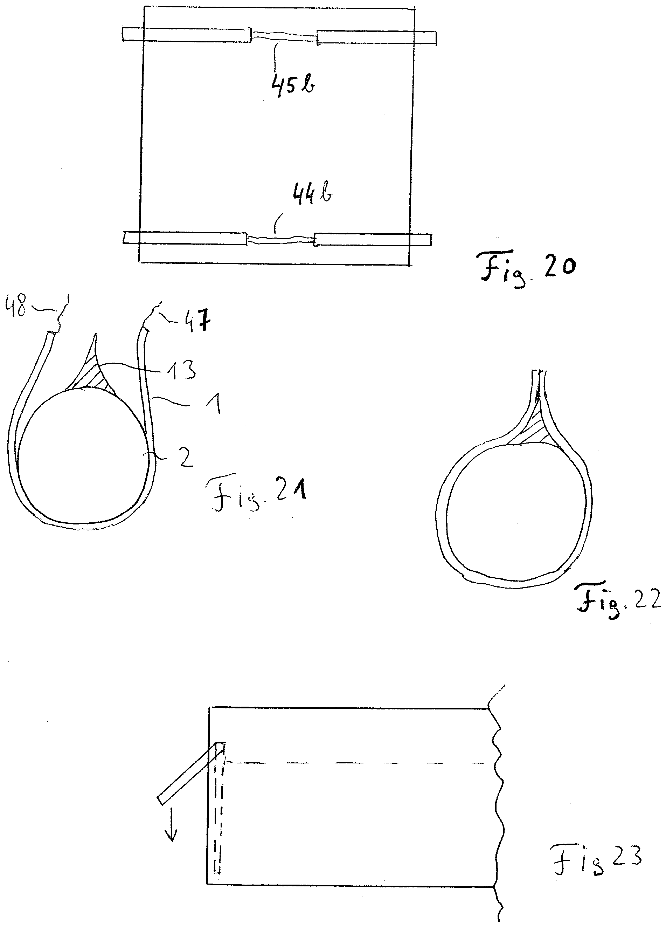

Figur 20 stellt schematisch den dadurch entstehenden Zustand dar, in dem Klebstreifen beziehungsweise Tranferkleber 44b und 45b im mittleren Bereich des Abdeckelements 1 freigelegt ist zur Vorfixierung des Körperteils.

Nach der Vorfixierung des Körperteils 2 entsteht die Situation, die in der Figur 21 dargestellt ist. Ein Manschettenkörper 13 wird auf das Körperteil 2 aufgelegt oder aufgeklebt und das folienförmige Abdeckelement 1 wird zu beiden Seiten mehr oder weniger symmetrisch nach oben gezogen, um das Körperteil 2 einzuhüllen. Danach können entsprechende Passelemente, die in der Figur 21 nicht eigens dargestellt sind, passend zusammengefügt werden, um eine Vorausrichtung zu erreichen.

Die Enden der Abziehelemente sind in der Figur 21 im oberen Bereich dargestellt und mit 47, 48 bezeichnet.

Nach dem festen Andrücken des Abdeckelementes 1 an das Körperteil und den Manschettenkörper wird die in der Figur 22 dargestellte Form erreicht. Sodann können Abziehelemente schräg, beispielsweise in einem Winkel von weniger als 90°, beislielweise 45° vom Rand der Abdeckung abgeknickt und schräg zwischen dem Abdeckelement 1 und dem Körperteil hervorgezogen werden. Der oder die Klebstreifen unter den entsprechenden Abziehelementen werden damit auf dem Abdeckelement freigelegt und dies wird gasdicht mit dem Körperteil 2, dem Manschettenkörper und sich selbst verbunden.

Es kann auch eine anfängliche, vorbereitete Abwinkelung von Abziehelementen in einem Winkel von weniger als 90°, 45° oder einem ähnlichen Winkel über die Kontur des Abdeckelementes hinaus vorgesehen sein, um den Beginn des Abziehens zu erleichtern.

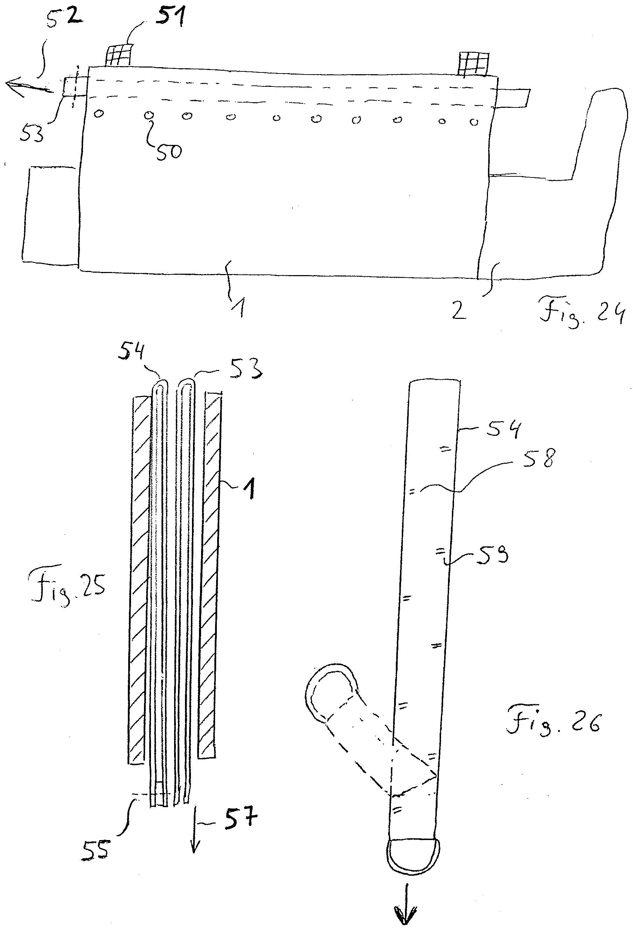

Die Figur 24 zeigt eine Seitenansicht eines Körperteils 2, in diesem Fall eines Beins, mit einem Abdeckelement 1, bei dem bereits die in Umfangsrichtung des Beins verlaufenden Abziehelemente 47a,48a,49a und 50a weggezogen und dort gasdichte Verbindungen zwischen dem Abdeckelement und dem Bein sowie dem Manschettenkörper gebildet sind. Zuletzt bleibt die Verbindung der beiden Enden des Abdeckelementes 1 am oberen Rand herzustellen, wo bereits eine Vorfixierung durch die schematisch dargestellten Passelemente, entweder beispielsweise Druckknöpfe 50 oder überstehende Laschen beispielsweise in Klettverschlussform 51, realisiert ist. Dort liegen, in Figur 24 schematisch durch gestrichelte Linien dargestellt, die Abziehelemente 53,54.

In einem folgenden Schritt kann in Richtung des Pfeils 52 an je einer Lasche 53 gezogen werden, um die entsprechenden Abziehstreifen zu entfernen.

In der Figur 25 ist der Abziehstreifen beziehungsweise ein Paar von Abziehstreifen 53,54 detaillierter in einer Ansicht gemäß Figur 24, dort von oben gesehen dargestellt. Die einzelnen Abziehstreifen 53, 54 sind jeweils einmal gefaltet und liegen somit in je 2 Lagen. An dem Ende, an dem sie herausgezogen werden, können die dort an sich freien Enden jeweils ebenfalls miteinander verbunden sein, beispielsweise in lösbarer Form durch eine Prägung, durch Kleben oder Heften. Diese Verbindung wird vor dem Herausziehen oder während des Herausziehens, ebenso wie die entlang der Länge vorgesehenen, in der Figur 26 dargestellten Verbindungen 58, 59, gelöst. Die endseitigen Verbindungen können beispielsweise durch Abreißen der Enden entlang einer Perforierung 55 in Figur 25 gelöst werden. Die Verbindungen zwischen den 2 Lagen sind vorteilhaft zur Stabilisierung der Abziehelemente vorgesehen, um eine Verschiebung zu vermeiden. Nach der Lösung der Verbindungen können die Abziehelemente 53, 54 in Richtung der Pfeile 56,57 gerade herausgezogen werden, so dass die beiden einander zugewandten, mittleren Lagen der Elemente 53,54 gemeinsam oder einzeln verschoben und die gesamten Elemente jeweils eingerollt werden. Es ergibt sich somit keine Scherbewegung eines Abziehelementes gegenüber einer Kleberschicht, so dass das Herausziehen leicht durchführbar ist.
Die Abziehelemente können auch schräg, das heißt in der Darstellung der Figur 25 innerhalb der Zeichenebene seitwärts beispielsweise um einen Winkel von 30- 45 Grad geneigt, herausgezogen werden, wie in Figur 26 gestrichelt angedeutet.

Die Abziehelemente können auch schon so vorbereitet wein, dass entsprechende Abziehenden seitlich über die Kontur des Abdeckelementes schräg hinausragend herausgeführt sind.

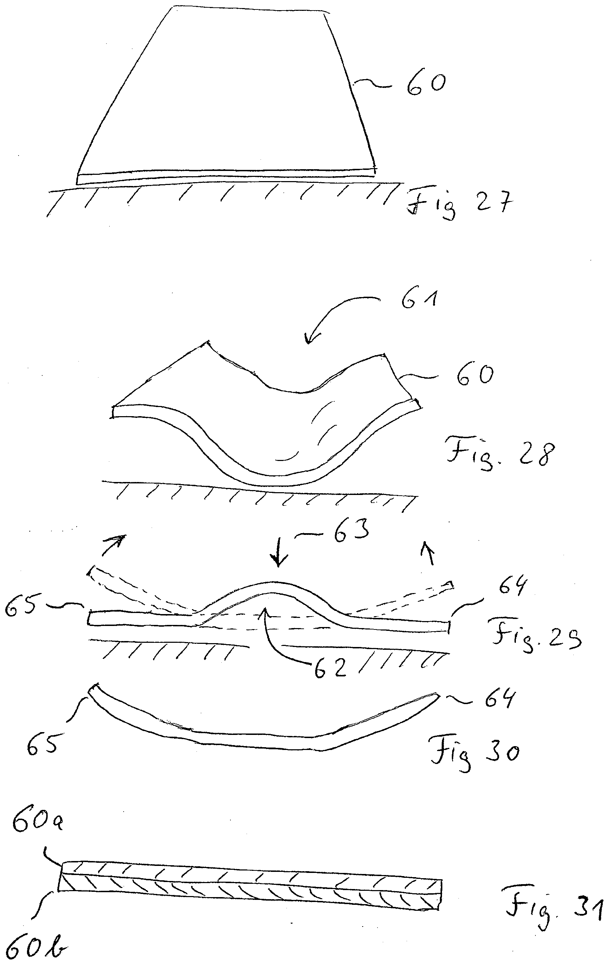

In Figur 27 ist ein aus einem geschlossenporigen Schaumstoff bestehendes Flachmaterial 60 in wesentlichen in rechteckiger Form dargestellt.

Figur 28 zeigt das Flachmaterial aus Figur 27 nach einer Behandlung, bei der eine Rinnenform 61 in das Flachmaterial eingebracht wird. Dies kann beispielsweise durch Tiefziehen einer flachen Form, durch Pressen oder schon im Herstellungsprozess bei der Herstellung des Schaumstoffs in einer Form geschehen. Die eingebrachte Form kann eine einfache Rinnenform, das heißt eine teilzylindrische Form oder auch eine kalottenförmige Muldenform oder die Nachahmung einer anatomischen Form eines Körpers oder eines Körperteils sein.

Das Material ist derart ausgewählt und in seiner Dicke so bemessen, dass die Abdeckung auch gegen die Wirkung der Gravitationskraft so weit in der in Figur 28 dargestellten Form bleibt, dass die hochstehenden Ränder stabil von einer Unterlage, auf die die Abdeckung gelegt wird, abstehen.

Die Rinne kann wie in Figur 29 gezeigt, auch umgekehrt in die Abdeckung eingebracht werden beziehungsweise die Verwendung der Abdeckung kann derart sein, dass die Öffnung der Rinne bei der Ablage auf einer flachen Unterlage nach unten weist. Die Rinne ist in der Figur 29 mit 62 bezeichnet. In der durchgezogenen Form ist die Abdeckung in Figur 29 unbelastet dargestellt, wobei die beiden einander gegenüberliegenden Ränder 64, 65 auf einer flachen Unterlage aufliegen.

Wird die Abdeckung durch eine Belastung in Richtung des Pfeils 63 durch einen auf ihr abgelegten Gegenstand belastet, so werden, wie in der gestrichelten Form dargestellt, die Ränder 64, 65 angehoben während der Mittelteil der Abdeckung, in dem sich die Rinne 62 befindet, flach auf die Unterlage gedrückt wird.

Dieser Zustand ist in der Figur 30 in durchgezogener Linienform dargestellt.

Die besondere Form der Abdeckung, die von der flachen, ebenen Form abweicht und die beim Anlegen eines gasdichten Wundverbandes eine einfache Handhabung erlaubt, kann außer durch die oben erwähnten Verformungsmethoden auch durch besondere Behandlung eines ein- oder zweischichtigen Flachmaterials erreicht werden, das beispielhaft in der Figur 31 dargestellt ist. Dort sind zwei Schichten 60a, 60b dargestellt. Beispielsweise kann die Schicht 60a ein Material sein, das bei Wärmeeinwirkung schrumpft, so dass bei gleich bleibender Ausdehnung des Materials 60b die gesamte Abdeckung gekrümmt wird, sobald das Material 60a kontrahiert wird. Dies kann durch Temperatureinwirkung beispielsweise auch schon bei Körpertemperatur oder durch Einwirkung von Strahlung oder durch eine chemische Reaktion erfolgen.

Beispielsweise ist denkbar, dass das Flachmaterial auf eine Unterlage gelegt wird, dass darauf ein Körperteil abgelegt wird und dass nach dem Ablegen des Körperteils mittels eines üblichen Haartrockners Wärme von oben auf die Abdeckung aufgebracht und damit ein Schrumpfungsprozess auf der Innenseite, auch bei homogener Ausführung des Flachmaterials eingeleitet wird, der zum Hochbiegen der Ränder der Abdeckung führt.

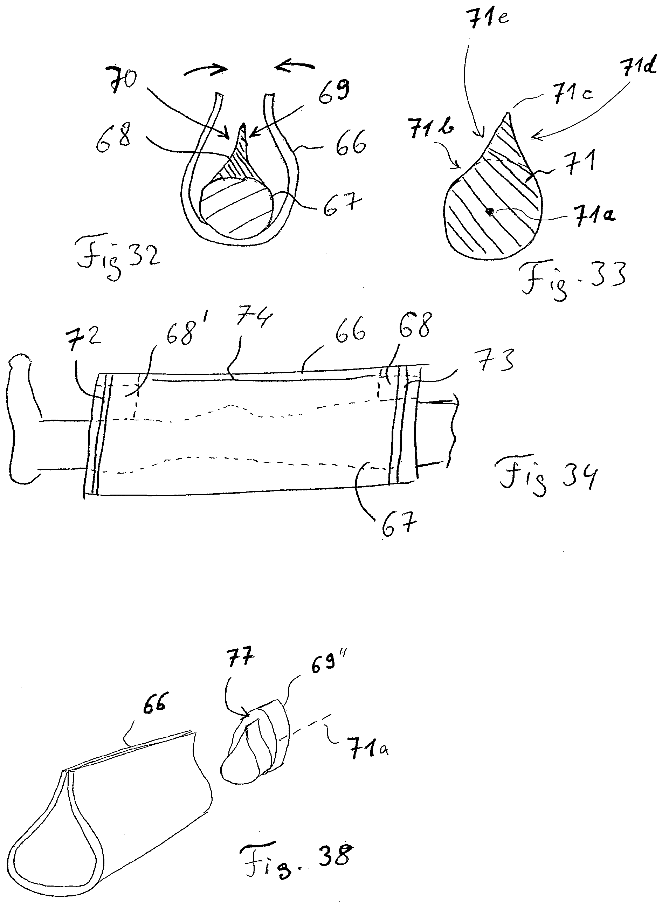

Figur 32 zeigt einen Querschnitt durch ein Abdeckelement 66 sowie ein schematisch dargestelltes Bein 67 und einen Manschettenkörper 68, der im Bereich des Querschnittes auf das Bein 67 gasdicht aufgesetzt beziehungsweise aufgeklebt ist. Der Manschettenkörper weist zwei konkave, spitz aufeinander zulaufende Seitenflächen 69, 70 auf, an die das Abdeckelement 66 anlegbar und anklebbar ist. Die beiden freien Enden des Abdeckelementes können dann oberhalb des Manschettenkörpers 68 zusammentreffen, ohne dass ein Zwickelraum gebildet wird, der beim Abdichten Schwierigkeiten machen könne.

Figur 33 zeigt im Querschnitt einen Abschlusskörper 71, der im Ganzen eine ähnliche Gestalt aufweist wie im Querschnitt das Bein 67 zusammen mit dem Manschettenkörper 68. Der Abschlusskörper weist eine Längsachse 71a, eine Mantelfläche 71b, einen Kantenbereich 71c und zwei konkave Prismenflächen 71d, 71e auf.

Wenn das Abdeckelement an einem Ende an einem Abschlusselement 71 und an seinem anderen Ende an einer Extremität 67 und einem Manschettenkörper 68 anliegt, so kann insgesamt eine symmetrische Gestaltung erreicht werden.

Figur 34 zeigt eine Ansicht eines Abdeckelementes 66 von der Seite, wobei schematisch das Bein 67 sowie der Manschettenkörper 68 angedeutet sind sowie ein weiterer Manschettenkörper 68' im Bereich des Fußes. Der Transferkleber ist durch Linien 72, 73, 74 schematisch angedeutet.

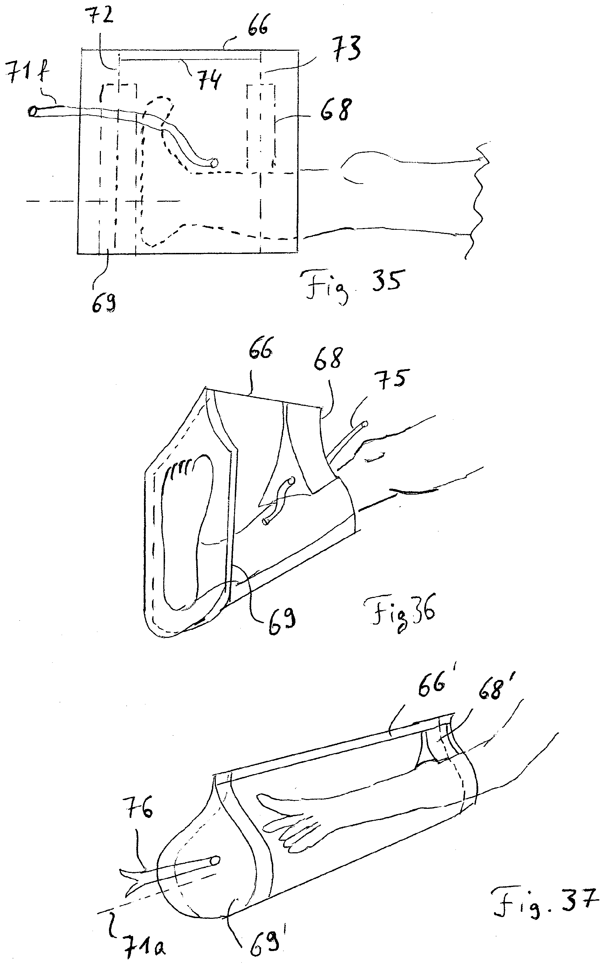

Figur 35 zeigt im Gegensatz dazu ein Abdeckelement 66, das einen Unterschenkel und einen Fuß umgibt. Das Flachmaterial kann identisch sein mit demjenigen eines Abdeckelementes, das gemäß Figur 34 eingesetzt wird.

Gemäß Figur 35 ist das Abdeckelement 66 auf einer Seite mittels eines Manschettenkörpers 68 an dem Unterschenkel des Patienten abgedichtet während es am anderen Ende über den Fuß hinausragt und an einem Abschlusselement 69 gasdicht anliegt und angeklebt ist. Die Streifen des Transferklebers sind wiederum in Figur 35 mit den Bezugszeichen 72, 73 und 74 bezeichnet. Das Abschlusselement 69 kann eine Laufsohle bilden und beispielsweise auch mehrschichtig ausgebildet sein. Es kann auch von einer Vakuumleitung 71f durchsetzt sein.

In der Figur 36 ist die Konstellation aus Figur 35 dreidimensional schematisch dargestellt, wobei das Abdeckelement 66 im Bereich des Manschettenkörpers 68 durchbrochen ist. Im Bereich des Manschettenkörpers 68 ist ein Vakuumschlauch 75 gasdicht durchgeführt, der schematisch dargestellt ist.

Figur 37 zeigt ein Abdeckelement 66', das eine Hand und einen Unterarm umgibt und im Bereich des Unterarms mittels eines Manschettenkörpers 68' abgedichtet ist. Stirnseitig vor der Hand befindet sich ein Abschlusskörper 69', der ebenso geformt sein kann wie der Abschlusskörper 69 aus Figur 36, jedoch maßstabsgerecht verkleinert sein kann und der insgesamt etwa die Größe aufweisen kann wie der Unterarm des Patienten im Querschnitt gemeinsam mit dem Manschettenkörper 68'.

In dem Abschlusskörper 69 kann ein Manipulationsinstrument 76 befestigt sein, das dem Patienten das Ausführen bestimmter Aktivitäten erlauben kann.

Das Abschlusselement 69, 69' kann beispielsweise ebenso wie das Abdeckelement 66, 66' wenigstens teilweise aus einem geschlossenporigen Schaumstoff oder aus einem offenporigen und mit einer Abdeckschicht verschlossenen Schaumstoff bestehen. Es kann jedoch auch aus einem massiven Kunststoff, beispielsweise einem Gummi oder einem anderen Elastomer bestehen.

Ein Abschlusselement kann ebenso wie beispielsweise auch ein Manschettenkörper eine wieder verschließbare Öffnung zum Einbringen eines Endoskops zur Wundinspektion aufweisen.

Wie in Figur 38 gezeigt, kann das Abschlusselement auch einen umlaufenden Absatz 77 aufweisen, der zu einer mehrstufigen Ausführung des Abschlusselements führt, wobei der Absatz eine Stirnfläche als Anschlagfläche und zum Schutz des röhrenförmigen Flachmaterials bildet. Der Absatz ist vorteilhaft durch eine umlaufende, im Querschnitt rechtwinklige Ausnehmung gebildet.

### Aspekte der Erfindung

1. Einrichtung zum Absaugen eines Fluids aus einer gasdichten, flexiblen Abdeckung (1) eines Körperteils (2) eines Lebewesens mit einer Absaugöffnung (17) in der Abdeckung,
   gekennzeichnet durch wenigstens einen wenigstens abschnittsweise strangförmigen Absaugkörper (31,32,33,42,43), der zwischen der Abdeckung und dem Körperteil entlang der Oberfläche des Körperteils verläuft, der wenigstens teilweise aus einem kompressiblen porösen Stoff, insbesondere einem Schaumstoff, besteht und innerhalb dessen ein durchgehender Absaugpfad gebildet ist.
2. Einrichtung zum Absaugen eines Fluids aus einer gasdichten, flexiblen Abdeckung (1) eines Körperteils (2) eines Lebewesens mit einer Absaugöffnung (17) in der Abdeckung,
   gekennzeichnet durch einen Absaugkörper (31,32,33,42,43), der wenigstens teilweise aus einem kompressiblen porösen Stoff, insbesondere einem Schaumstoff, besteht, der das abzudeckende Körperteil in Teilbereichen abdeckt, die durch die Geometrie des Absaugkörpers bestimmt sind und innerhalb dessen ein durchgehender Absaugpfad zur Absaugöffnung gebildet ist.
3. Einrichtung nach Aspekt 1 oder 2,
   wobei der Absaugkörper (31,32,33,42,43) wenigstens teilweise aus einem offenporigen Schaumstoff gebildet ist.
4. Einrichtung nach Aspekt 1, 2 oder 3,
   wobei der/ein Absaugkörper (31,32,33,42,43) wenigstens abschnittsweise im Querschnitt rund oder rechteckig ausgebildet ist.
5. Einrichtung nach einem der Aspekte 1 bis 4,
   wobei der/ein Absaugkörper (31,32,33,42,43) aus einem schichtförmigen Vlies (38) durch Entfernen von Teilflächen des Vlieses gebildet ist.
6. Einrichtung nach Aspekt 1 oder einem der folgenden,
   wobei der/ein Absaugkörper (31,32,33,42,43) wenigstens in den Teilbereichen (41) seiner Oberfläche, die auf dem Körperteil (2) aufliegen, gasdicht ausgebildet ist.
7. Einrichtung nach Aspekt 1 oder einem der folgenden,
   wobei der/ein Absaugkörper (31,32,33,42,43) einen oder mehrere strangförmige oder streifenförmige Abschnitte aufweist, die sich verzweigen.
8. Einrichtung nach Aspekt 1 oder einem der folgenden,
   wobei der/ein Absaugkörper (31,32,33,42,43) einen oder mehrere strangförmige oder streifenförmige Abschnitte aufweist, die in einer Gitterstruktur miteinander verbunden sind.
9. Einrichtung nach Aspekt 1 oder einem der folgenden,
   wobei ein Absaugpfad die Absaugöffnung mit einem Absaugkissen (36,37) in einem Wundbereich (34,35) verbindet.
10. Gasdichte Abdeckung (1,66) für einen Körper oder ein Körperteil (2) eines Lebewesens mit einem flexiblen gasdichten folienartigen Flachmaterial(1,60,60a,60b), das zumindest abschnittsweise derart vorbereitet, insbesondere vorgeformt ist, dass, wenn die Abdeckung auf einer flachen Unterlage angeordnet und mit ausreichender Kraft gegen die Unterlage gedrückt wird, insbesondere wenn ein ausreichend schwerer Gegenstand auf der Abdeckung abgelegt wird, die Ränder(64,65) der Abdeckung an zwei einander gegenüber liegenden Seiten von der Unterlage abheben.
11. Gasdichte Abdeckung(1,66) für einen Körper oder ein Körperteil eines Lebewesens mit einem flexiblen gasdichten folienartigen Flachmaterial(1,60,60a,60b), das zumindest abschnittsweise rinnenförmig vorgeformt ist.
12. Gasdichte Abdeckung nach Aspekt 10 oder 11,
   wobei das Flachmaterial(1,60,60a,60b) abschnittsweise muldenartig vorgeformt ist.
13. Gasdichte Abdeckung nach einem der Aspekte 10 bis 12, wobei die Abdeckung (1,66) durch thermische Behandlung eines Flachmaterials(1,60,60a,60b) vorgeformt ist.
14. Gasdichte Abdeckung nach einem der Aspekte 10 bis 13, dadurch gekennzeichnet,
   dass die Abdeckung durch Pressen oder Tiefziehen eines Flachmaterials(1,60,60a,60b) vorgeformt ist.
   Gasdichte Abdeckung insbesondere auch nach einem der Aspekte 10 bis 14, für einen Körper oder ein Körperteil(2) eines Lebewesens mit einem flexiblen gasdichten folienartigen Flachmaterial(1,60,60a,60b), das zumindest abschnittsweise aus zwei Schichten besteht, von denen eine erste andere Verformungseigenschaften aufweist als die zweite.
15. Gasdichte Abdeckung nach Aspekt 14,
   wobei eine erste (60a) der Schichten durch Einwirkung von Wärme, Kälte, Strahlung oder einem mit der Schicht wechselwirkenden Fluid kontrahierbar ist.
16. Gasdichte Abdeckung nach einem der Aspekte 10 bis 15, wobei die Abdeckung (1,66) mit gasdichten Klebestreifen zur Abdichtung beim Verschließen versehen ist.
17. Gasdichte Abdeckung nach Aspekt 16,
   wobei die Klebestreifen durch Abziehelemente bis zum Zusammenfügen abgedeckt sind.
18. Gasdichte Abdeckung nach Aspekt 10 oder einem der folgenden,wobei an den jeweiligen miteinander zu verbindenden Rändern der Abdeckung Passelemente zur Vorpositionierung vor der Fixierung des Abdeckelementes vorgesehen sind.
19. Abdeckelement für den gasdichten Abs1chluss eines Körperteils mit einem Folienelement (1), das um das Körperteil (2) herum gasdicht zusammenfügbar ist gekennzeichnet durch
   wenigstens einen Manschettenkörper (13), der als Prisma mit drei im Querschnitt des Prismas spitzwinklig aufeinanderstoßenden Seitenflächen ausgebildet ist.
20. Abdeckelement nach Aspekt 19,
   wobei wenigstens eine der Seitenflächen im Querschnitt des Prismas (13,14) konkav ausgebildet ist.
21. Abdeckelement nach Aspekt 20
   wobei wenigstens zwei, insbesondere alle drei Seitenflächen des Prismas (13,14) im Querschnitt des Prismas konkav ausgebildet sind.
22. Abdeckelement nach Aspekt 19 oder einem der folgenden, wobei das Prisma/die Prismen (13,14) aus einem geschlossenporigen Schaumstoff besteht/bestehen.
23. Abdeckelement nach Aspekt 19 oder einem der folgenden, wobei das/die Prismen (13,14) mit einem Kleber (21) beschichtet sind.
24. Abdeckelement nach Aspekt 23,
   wobei die Beschichtung (21) an den Kanten, an denen die Seitenflächen des jeweiligen Prismas (13,14) aneinander angrenzen, über die Kanten hinaus ragt.
25. Abdeckelement nach Aspekt 19 oder einem der folgenden, wobei das wenigstens eine Prisma (13,14) eine gasdichte Durchführung für Fluide aufweist.
26. Abdeckelement nach Aspekt 25,
   wobei die Durchführung einen Gasschlauch (17) zum Absaugen von Gas aufweist.
27. Abdeckelement nach Aspekt 26,
   wobei der Gasschlauch (17) an seinem Ende innerhalb des durch das Abdeckelement abgeschlossenen Innenraums mantelseitige Öffnungen aufweist.
28. Manschettenkörper (13,14), ausgebildet als dreikantiges Prisma insbesondere mit konkaven Seitenflächen, bestehend aus einem geschlossenporigen Schaumstoff zur Verwendung bei einem Abdeckelement nach einem der Aspekte 19 bis 27.
29. Abdeckelement für die gasdichte Abdeckung eines Körperteils mit einem Folienelement, das um das Körperteil herum zu dessen Abdeckung gasdicht zusammenfügbar ist, wobei das Folienelement eine Längsrichtung aufweist, die im wesentlichen parallel zur Längsachse des abzudeckenden Körperteils auszurichten ist sowie quer dazu verlaufende Querrichtungen, gekennzeichnet durch wenigstens ein, insbesondere 2 Kleberstreifen, die in einer Querrichtung verlaufend auf dem Folienelement haften und bezüglich ihrer Länge in einen Vorfixierabschnitt und beiderseits des Vorfixierabschnittes je einen Randabschnitt geteilt sind sowie durch ein oder mehrere, den oder die Kleberstreifen abdeckende Abziehelemente in Form von ebenfalls in Querrichtung verlaufenden Abziehstreifen, wobei der / die Abziehstreifen dazu eingerichtet ist/sind, in dem Vorfixierabschnitt, vom Kleberstreifen abgezogen zu werden, während der erste Randabschnitt und der zweite Randabschnitt des Kleberstreifens weiterhin von Teilen eines Abziehstreifens bedeckt bleibt.
30. Abdeckelement nach Aspekt 29, dadurch gekennzeichnet, dass das/die Abziehelemente im Bereich zwischen dem ersten und dem zweiten Randabschnitt wenigstens eine, insbesondere zwei Unterbrechung(en) oder eine oder zwei zur Unterbrechung vorbereitete Stelle(n) aufweist(aufweisen).
31. Abdeckelement nach Aspekt 29, wobei das/die Abziehelemente auf jeder Seite des Vorfixierabschnittes jeweils zwischen diesem und einem Randabschnitt insgesamt wenigstens eine, insbesondere beiderseits des Vorfixierabschnittes zwei Unterbrechung(en) oder jeweils eine oder zwei zur Unterbrechung vorbereitete Stelle(n) aufweist(aufweisen).
32. Abdeckelement nach Aspekt 29, 30 oder 31, wobei wenigstens ein Abziehelement einen Greifabschnitt aufweist, der in wenigstens einer Richtung von der Längsverlaufsrichtung des Klebstreifens, den das Abziehelement abdeckt, absteht und das vor und/oder während und/oder nach der Zusammenfügung des Abdeckkörpers zum Abziehen greifbar ist.
33. Abdeckelement nach Aspekt 29, 30, 31 oder 32, wobei wenigstens eines der Abziehelemente aus wenigstens zwei gegeneinander verschiebbaren Lagen besteht.
34. Abdeckelement nach Aspekt 33, wobei die beiden Lagen in wenigstens einem Bereich lösbar miteinander verbunden sind.
35. Abdeckelement nach Aspekt 34, dadurch gekennzeichnet, dass die beiden Lagen in einem Endabschnitt, der insbesondere über das Abdeckelement hinausragt, miteinander verbunden sind.
36. Abdeckelement nach Aspekt 34 oder 35, wobei beiden Lagen entlang der Länge des Klebstreifens wenigstens abschnittsweise oder punktweise miteinander lösbar verbunden sind.
37. Abziehelement (15,16) insbesondere zur Verwendung mit einem Abdeckelement gemäß den vorangehenden Aspekten, zur Herstellung einer Fügeverbindung zwischen zwei einander zugewandten Fügeflächen (3,4) durch Herausziehen des Abziehelementes zwischen den Fügeflächen,
   gekennzeichnet durch
   ein erstes und ein zweites flexibles Haftstreifenelement (23,24,29,30), die jeweils bezüglich ihrer Längsrichtungen parallel zueinander verlaufen und zwischen sich einen Zwischenraum (26) bilden, in dem ein mit beiden Haftstreifen verbundenes Zugelement verschiebbar angeordnet ist.
38. Abziehelement nach Aspekt 9,
   wobei die Haftstreifenelemente (23,24) deckungsgleich aufeinander liegen.
39. Gasdichte Abdeckung (66) für ein Körperteil(2, 67)) eines Lebewesens mit einem flexiblen, gasdichten, folienartigen Flachmaterial (1,60,60a,60b), das um das Körperteil röhrenartig herumlegbar und gasdicht zusammenfügbar ist, gekennzeichnet durch ein gasdichtes Abschlusselement(69,69',69",71), das an einem Ende eine offene Stirnseite der röhrenartigen Abdeckung gasdicht verschließt.
40. Gasdichte Abdeckung nach Aspekt 39, dadurch gekennzeichnet, dass das Abschlusselement(69,69',69",71) eine erste Achse (71a) aufweist, die im montierten Zustand die Abdeckung im wesentlichen parallel zur Längsachse (71a) des röhrenartigen Abdeckelementes ist und entlang deren das Abschlusselement zumindest abschnittsweise eine gleichförmige Querschnittsfläche aufweist.
41. Gasdichte Abdeckung nach Aspekt 40,
   dadurch gekennzeichnet,
   dass die azimutal um die erste Achse verlaufende Mantelfläche des Abschlusselementes(69,69',69",71) bis auf einen einzigen Bereich (71c) des Umfangs abgerundet und kantenfrei ist.
42. Gasdichte Abdeckung nach Aspekt 39 oder einem der folgenden, wobei das Abschlusselement(69,69',69",71) an einer Stelle seines Umfangs einen prismatischen Ansatz mit zwei im Querschnitt spitzwinklig aufeinander zulaufenden Prismenflächen (71d,71e) aufweist.
43. Gasdichte Abdeckung nach Aspekt 42,
   wobei die spitzwinklig aufeinander zulaufenden Prismenflächen (71d,71e) jeweils konkav ausgebildet sind.
44. Gasdichte Abdeckung nach Aspekt 39 oder einem der folgenden,
   wobei das Abschlusselement(69,69',69",71) zur Anwendung an einem Bein vorgesehen ist und eine Laufsohle bildet.
45. Gasdichte Abdeckung nach einem der Aspekte 39 bis 44,

   wobei durch das Abschlusselement(69,69',69",71) stirnseitig ein Fluidschlauch (71f) gasdicht durchgeführt ist.
46. Gasdichte Abdeckung nach einem der Aspekte 39 bis 44,
   wobei in das Abschlusselement(69,69',69",71) stirnseitig ein Manipulationswerkzeug (76) eingebettet ist.
47. Gasdichte Abdeckung nach Aspekt 39 oder einem der folgenden, wobei das röhrenartig geformte Flachmaterial(1,60,60a,60b) an der Mantelseite des Abschlusselementes(69,69',69",71) mittels einer flexiblen Dichtsubstanz insbesondere eines Transferklebers gedichtet ist.
48. Gasdichte Abdeckung nach einem der Aspekte 39 bis 47, wobei das röhrenartig geformte Flachmaterial(1,60,60a,60b) an einer Stirnseite (77) des Abschlusselementes(69,69',69",71) dichtend anliegt, die im wesentlichen senkrecht zu seiner Längsachse (71a) verläuft.
49. Abdeckelement für den gasdichten Abschlusses eines Körperteils (2) mit einem Folienelement (1) und mit Fügeflächen (3,4), an denen Teile des Folienelementes zur Schaffung eines abgeschlossenen Innenraums gasdicht zusammenfügbar sind,
   gekennzeichnet durch
   wenigstens zwei Passelemente (5,6,7,8), die im Bereich der Fügeflächen an dem Folienelement (1) befestigt und zum gegenseitigen Ausrichten der Fügeflächen formschlüssig zusammenfügbar sind.
50. Abdeckelement nach Aspekt 49,
   wobei die Passelemente über den Rand des Folienelementes (1) hinaus ragen.
51. Abdeckelement nach Aspekt 49 oder 50,
   wobei der Bereich der Passelemente (5,6,7,8), in dem die formschlüssige Verbindung hergestellt wird, über den Rand des Folienelementes (1) hinaus ragt.
52. Abdeckelement nach Aspekt 49 oder einem der folgenden,
   wobei wenigstens eines der Passelemente (5,6,7,8) einen Handgriff (9) aufweist.
53. Abdeckelement nach Aspekt 49 oder einem der folgenden, wobei die Passelemente (5,6,7,8) nach dem Zusammenfügen der Fügeflächen (3,4) von dem Folienelement (1) abnehmbar sind.
54. Abdeckelement nach Aspekt 49 oder einem der folgenden, wobei Vorfixierelemente (10,11,12) zur Fixierung der Fügeflächen relativ zueinander vorgesehen sind.
55. Abdeckelement nach Aspekt 54,
   wobei die Vorfixierelemente (19,11,12) wenigstens ein Klettverbindungselement aufweisen.
56. Abdeckelement nach Aspekt 54 oder 55,
   wobei die Vorfixierelemente (10,11,12)von den Fügeflächen aus gesehen außerhalb des Innenraums gelegen sind.
57. Abdeckelement nach Aspekt 49 oder einem der folgenden, wobei zwischen den Fügeflächen (3,4) wenigstens ein Abziehelement(15,16) zum Freilegen der haftenden Fügeflächen vorgesehen ist.

**Bezugszeichenliste**

| | |
|---|---|
| 1 | Folienelement |
| 2 | Körperteil |
| 3, 4 | Fügeflächen |
| 5, 6, 7, 8 | Passelemente |
| 5a | Zapfen |
| 8a | Passlöcher |
| 9 | Passelement mit Handgriff |
| 10, 11, 12 | Vorfixierelemente |
| 13, 14 | Manschettenkörper |
| 15 | Abziehelement |
| 16 | Schlauch |
| 17 | Vakuumleitung |
| 18 | Vakuumconnector |
| 19, 20 | Anschlüsse |
| 21 | Klebstoff |
| 23, 24, 29, 30 | Haftstreifenelemente |
| 23a, 24a | Enden der Haftstreifenelemente |
| 25 | Zugelement |
| 26 | Zwischenraum |
| 27 | Zugrichtung |
| 28 | Silikonschicht |
| 31, 32 | Randbereiche der Haftstreifenelemente |

## Patentansprüche

1. Abdeckelement für den gasdichten Abs1chluss eines Körperteils mit einem Folienelement (1), das um das Körperteil (2) herum gasdicht zusammenfügbar ist **gekennzeichnet durch**
wenigstens einen Manschettenkörper (13), der als Prisma mit drei im Querschnitt des Prismas spitzwinklig aufeinanderstoßenden Seitenflächen ausgebildet ist.

2. Abdeckelement nach Anspruch 1,
**dadurch gekennzeichnet,**
**dass** wenigstens eine der Seitenflächen im Querschnitt des Prismas (13,14) konkav ausgebildet ist.

3. Abdeckelement nach Anspruch 2
**dadurch gekennzeichnet,**
**dass** wenigstens zwei, insbesondere alle drei Seitenflächen des Prismas (13,14) im Querschnitt des Prismas konkav ausgebildet sind.

4. Abdeckelement nach Anspruch 1 oder einem der folgenden, **dadurch gekennzeichnet,**
**dass** das Prisma/die Prismen (13,14) aus einem geschlossenporigen Schaumstoff besteht/bestehen.

5. Abdeckelement nach Anspruch 1 oder einem der folgenden,
**dadurch gekennzeichnet,**
**dass** das/die Prismen (13,14) mit einem Kleber (21) beschichtet sind.

6. Abdeckelement nach Anspruch 5,
**dadurch gekennzeichnet,**
**dass** die Beschichtung (21) an den Kanten, an denen die Seitenflächen des jeweiligen Prismas (13,14) aneinander angrenzen, über die Kanten hinaus ragt.

7. Abdeckelement nach Anspruch 1 oder einem der folgenden,
**dadurch gekennzeichnet,**
**dass** das wenigstens eine Prisma (13,14) eine gasdichte Durchführung für Fluide aufweist.

8. Abdeckelement nach Anspruch 7,
**dadurch gekennzeichnet,**
**dass** die Durchführung einen Gasschlauch (17) zum Absaugen von Gas aufweist.

9. Abdeckelement nach Anspruch 8,
**dadurch gekennzeichnet,**
**dass** der Gasschlauch (17) an seinem Ende innerhalb des durch das Abdeckelement abgeschlossenen Innenraums mantelseitige Öffnungen aufweist.

10. Manschettenkörper (13, 14), ausgebildet als dreikantiges Prisma mit konkaven Seitenflächen, bestehend aus einem geschlossenporigen Schaumstoff zur Verwendung bei einem Abdeckelement nach einem der Ansprüche 1 bis 9.

## Claims

1. Covering element for closing off a body part in a gas-tight manner, having a foil/film element (1) which is joinable in a gas-tight manner around the body part (2), **characterized by** at least one sleeve body (13) which is configured as a prism having three lateral faces that abut one another at acute angles in the cross section of the prism.

2. Covering element according to Claim 1, **characterized in that** at least one of the lateral faces in the cross section of the prism (13, 14) is configured so as to be concave.

3. Covering element according to Claim 2, **characterized in that** at least two, in particular all three lateral faces of the prism (13, 14) in the cross section of the prism are configured so as to be concave.

4. Covering element according to Claim 1 or one of the following claims, **characterized in that** the prism/the prisms (13, 14) is/are composed of a foam material having closed pores.

5. Covering element according to Claim 1 or one of the following claims, **characterized in that** the prism/prisms (13, 14) is/are covered with an adhesive (21).

6. Covering element according to Claim 5, **characterized in that** the coating (21) on those edges on which the lateral faces of the respective prism (13, 14) are mutually adjacent protrudes beyond the edges.

7. Covering element according to Claim 1 or one of the following claims, **characterized in that** the at least one prism (13, 14) has a gas-tight passage for fluids.

8. Covering element according to Claim 7, **characterized in that** the passage has a gas tube (17) for suctioning gas.

9. Covering element according to Claim 8, **characterized in that** the gas tube (17) on the end thereof within the interior that is closed off by the covering element has jacket-side openings.

10. Sleeve body (13, 14) configured as a triangular prism having concave lateral faces, composed of a foam material having closed pores, for use in a covering element according to one of Claims 1 to 9.

## Revendications

1. Élément couvrant pour fermer de façon étanche aux gaz une partie de corps avec un élément en film (1) assemblé autour de la partie de corps (2) de façon étanche aux gaz, **caractérisé par** la présence d'au moins un corps de manchon (13) réalisé sous la forme d'un prisme doté de trois surfaces latérales se rencontrant selon un angle aigu dans la section transversale du prisme.

2. Élément couvrant selon la revendication 1, **caractérisé en ce qu'**au moins une des surfaces latérales est réalisée selon une forme concave dans la section transversale du prisme (13, 14).

3. Élément couvrant selon la revendication 2, **caractérisé en ce qu'**au moins deux, notamment les trois surfaces latérales du prisme (13, 14), sont réalisées selon une forme concave dans la section transversale du prisme.

4. Élément couvrant selon la revendication 1 ou l'une quelconque des revendications suivantes, **caractérisé en ce que** le ou les prismes (13, 14) se composent d'une mousse à pores fermés.

5. Élément couvrant selon la revendication 1 ou l'une quelconque des revendications suivantes, **caractérisé en ce que** le ou les prismes (13, 14) sont enduits de colle (21).

6. Élément couvrant selon la revendication 5, **caractérisé en ce que** le revêtement (21) ressort au-delà des arêtes, au niveau des arêtes au niveau desquelles les surfaces latérales du prisme (13, 14) respectif se jouxtent les unes les autres.

7. Élément couvrant selon la revendication 1 ou l'une quelconque des revendications suivantes, **caractérisé en ce que** l'au moins un prisme (13, 14) comporte un passage pour fluides étanche aux gaz.

8. Élément couvrant selon la revendication 7, **caractérisé en ce que** le passage comporte un tuyau flexible de gaz (17) pour aspirer les gaz.

9. Élément couvrant selon la revendication 8, **caractérisé en ce que** le tuyau flexible de gaz (17) comporte au niveau de son extrémité des ouvertures du côté d'enveloppe, à l'intérieur de l'espace intérieur fermé par l'élément couvrant.

10. Corps de manchon (13, 14) réalisé sous la forme d'un prisme triangulaire avec des surfaces latérales concaves, composé d'une mousse à pores fermés à utiliser dans un élément couvrant selon l'une quelconque des revendications 1 à 9.
